# EUROPEAN PATENT APPLICATION

(11) **EP 4 434 478 A1**
(43) Date of publication of application: **25.09.2024**
(21) Application number: 23163740.6
(22) Date of filing: 23.03.2023
(51) Int. Cl.: A61B 17/29

(54) **A SURGICAL SYSTEM, AN ACTUATING DEVICE FOR ACTUATING A SURGICAL INSTRUMENT, AND A SURGICAL INSTRUMENT THEREFORE**

(71) Applicant: Mao, Zhangfan, Wuhan, Hubei 430010 (CN); Hu, Heng, 63533 Mainhausen (DE)
(72) Inventor: Mao, Zhangfan, Wuhan, Hubei 430010 (CN); Hu, Heng, 63533 Mainhausen (DE)
(74) Representative: Staudt, Armin Walter

(57) **Abstract**

Known surgical systems comprise a surgical instrument, and an actuating device for actuating the surgical instrument. The actuating device has a connector in which a hollow channel extends along a longitudinal axis between a distal end and a proximal end, wherein the distal end is provided with a first coupling means configured to be coupled to a first mating coupling means on the surgical instrument. The hollow channel of the connector axially movably receives a wire with a distal wire end and a proximal wire end, said distal wire end being provided with a second coupling means configured to be connected to a second mating coupling means on the surgical instrument, wherein said first coupling means being configured to be connectable to said first mating coupling means by rotation of said connector about its longitudinal axis in a first rotational direction. In order to specify an easy-to-use surgical system that further minimizes patient injury, thereby reducing the need for any surgical follow-up, it is suggested that said second coupling means is configured to be connectable to said second mating coupling means by rotation of said wire about said wire longitudinal axis in a second rotational direction opposite to said first rotational direction and in that the actuating device comprises a locking means which is movable between an open position and a closed position, the open position permitting relative rotatability of wire and connector in the first rotational direction and the second rotational direction and relative displaceability in the axial direction, and when said first coupling means is connected to said first mating coupling means and said second coupling means is connected to said second mating coupling means, the closed position preventing relative rotatability of wire and connector.

## Description

### TECHNICAL FIELD

The present disclosure relates in general to the field of surgical systems and instruments for multi-incision minimally invasive surgery, e.g., surgical instruments for thoracoscopic or laparoscopic surgery. These are also referred to as surgical instruments for intracavitary surgery or endoluminal surgery.

In particular the present disclosure relates to surgical systems, comprising a surgical instrument, and an actuating device for actuating the surgical instrument, the actuating device comprising a connector having a connector longitudinal axis in which a hollow channel extends along the longitudinal axis between a distal end and a proximal end, wherein the distal end is provided with a first coupling means configured to be coupled to a first mating coupling means on the surgical instrument, wherein said hollow channel axially movably receives a wire having a longitudinal wire axis essentially parallel with the connector longitudinal axis, and said wire having a distal wire end and a proximal wire end, said distal wire end being provided with a second coupling means configured to be connected to a second mating coupling means on the surgical instrument, said first coupling means being configured to be connectable to said first mating coupling means by rotation of said connector about said connector longitudinal axis in a first rotational direction.

The present invention further relates to an actuating device for actuating a surgical instrument, the actuating device comprising a connector having a connector longitudinal axis in which a hollow channel extends along the longitudinal axis between a distal end and a proximal end, wherein the distal end is provided with a first coupling means configured to be coupled to a first mating coupling means on the surgical instrument, wherein said hollow channel axially movable receives a wire having a longitudinal wire axis essentially parallel with the connector longitudinal axis, and said wire having a distal wire end and a proximal wire end, said distal wire end being provided with a second coupling means configured to be connected to a second mating coupling means on the surgical instrument, said first coupling means being configured to be connectable to said first mating coupling means by rotation of said connector about said connector longitudinal axis in a first rotational direction.

Finally, the present invention relates to a surgical instrument for a surgical system or an actuating device.

### STATE OF THE ART

In order to minimize the trauma associated with a surgery and to reduce the formation of scars, minimally invasive surgery is often used. Instead of making one large incision on the body surface of the patient, it is advantageous to make several, but smaller incisions and to provide them with a trocar through which one or more surgical instruments can be inserted in the patient's body. This approach is also referred to as multi-incision minimally invasive surgery.

EP 3 777 650 A1 describes a system for multi-incision minimally invasive surgery that can be disassembled and reassembled in the patient's body. For this purpose, the system is divided into two parts, one part being an operating device and the other part being a surgical instrument. The operating device is equipped with connector in form of a hollow needle which can be used for puncturing the patient's body. As the outer diameter of the hollow needle is less than 5 mm, the hollow needle can be removed from the body and the piercing point can recover on its own and there is no need to suture. The surgical instrument, on the other hand, is much larger and therefore requires a larger incision in the patient's body. Thus, one larger incision and a trocar is nevertheless necessary. The surgical instrument is placed in the patient's body using this trocar and the operating device is connected to the surgical instrument via the hollow needle. For this purpose, the hollow needle is equipped with a connecting part, e.g., with a clamping groove, a guide groove or an external thread. After the hollow needle is fixedly connected to the surgical instrument, the surgeon causes the pull line to pass through the hollow needle in order to control the surgical instrument by controlling the pull line outside the patient's body.

### TECHNICAL PROBLEM

However, passing the pull line through the hollow needle, is laborious and time-consuming. This is particularly true if the hollow needle has a small outer diameter as required for minimally invasive surgery. For example, the outer diameter of the hollow needle is less than 5 mm or even less than 3 mm.

It is an object of the present invention to provide an easy-to-use surgical system for minimizing patient injury and reducing the need for any surgical follow-up, that can be conveniently assembled in the patient's body. It is particularly desirable that the outer diameter of the hollow needle is so small that the wound does not need to be sutured. This is the case, for example, with an outer diameter of the hollow needle of less than 3mm, in particular 2.5mm or less.

It is a further object of the invention to provide an easy-to-use actuating device for actuating a surgical instrument, minimizing patient injury and reducing the need for any surgical follow-up, that can be conveniently assembled with surgical instrument in the patient's body.

Furthermore, it is a further object of the invention to provide an easy-to-use surgical instrument, that can be conveniently assembled with an actuating device for actuating the surgical instrument in the patient's body.

### BRIEF DESCRIPTION OF THE INVENTION

With regard to the surgical system, the above-mentioned technical objective is achieved in that said second coupling means is configured to be connectable to said second mating coupling means by rotation of said wire about said wire longitudinal axis in a second rotational direction opposite to said first rotational direction, and in that the actuating device comprises a locking means which is movable between an open position and a closed position, the open position permitting relative rotatability of wire and connector in the first rotational direction and the second rotational direction and relative displaceability in the axial direction, and the closed position preventing relative rotatability of wire and connector when said first coupling means is connected to said first mating coupling means and said second coupling means is connected to said second mating coupling means.

In known surgical systems, the connection of the surgical instrument to the actuating device in the patient's body may be complex and time-consuming. For example, feeding the wire back through the hollow channel may be a tricky task, particularly when the hollow channel of the connector has a small diameter, as is generally desirable in terms of minimizing patient injury. Therefore, the present invention is based on the idea of simplifying and improving the connection of actuating device and surgical instrument in order to provide a surgical system that can be assembled easily in the patient's body even when the hollow channel diameter is small, e.g., smaller than 2 mm. Preferably, the connector is a needle type connecting rod.

According to the present invention, two modifications are suggested, one concerning the connection of the wire to the surgical instrument and the other concerning the type of connection mechanism of surgical instrument and actuating device.

The basis for the first modification is the allocation of the wire to the actuating device. One reason for problems when connecting surgical instrument and actuating device is that the wire is connected to the surgical instrument and has to be fed back through the connector of the actuating device. The smaller the diameter of the connector, the more difficult it is to feed the wire back through the connector. A smaller diameter of the connector makes this more difficult. According to the invention, the wire is a part of the actuating device. Preferably, the connector and/or the wire is detachably connected to the actuating device. The wire may be already placed in the hollow channel when puncturing the patient's body or it can be inserted into the hollow channel subsequently from a position outside the patient body. This is much easier. Preferably, the wire is retractable to the extent that it does not protrude from the of the hollow channel.

However, this means that not only the connector but also the wire must be connected to the surgical instrument in the patient's body. According to the invention, the wire is provided with a special second coupling means as well as the surgical instrument is provided with a second mating coupling means. Both the first coupling means and the second coupling means are coupled to their mating coupling means by a rotation. Such a rotational connection is easy to create in the patient's body. Preferably, the connector is configured to be fixedly connected with its tip to the first mating coupling of the surgical instrument in the patient's body.

However, in order to prevent unintentional loosening of the connection between actuating device and surgical instrument, a special connection mechanism is suggested according to the second modification.

A connection based on rotation has a connecting rotation direction. It has the disadvantage that it can loosen if it is subjected to rotary movements in the opposite direction to the connecting rotation direction. Here, the first coupling means of the connector is connectable to the first mating coupling means on the surgical instrument by rotation of the connector about the connector longitudinal axis in a first rotational direction. This connection can prevent a disconnection of the connector only when the connector is rotated in the first direction of rotation. Furthermore, the wire has a second coupling means which is connectable a second mating coupling means by rotation of the wire about the wire longitudinal axis in a second rotational direction opposite to the first rotational direction. This connection can prevent a disconnection of the wire only when the wire is rotated in the second direction of rotation. The fact that the wire and the connector have different connecting rotation directions hinders the disconnection of actuating device and surgical instrument, but cannot prevent it.

In order to nevertheless achieve a stable connection of the actuating device with the surgical instrument, according to the invention the actuating device comprises a locking means that in closed position prevents relative rotatability of wire and connector when the first coupling means is connected to the first mating coupling means and the second coupling means is connected to the second mating coupling means.

Furthermore, the locking means can be moved to an open position, permitting relative rotatability of wire and connector in the first rotational direction and the second rotational direction and relative displaceability in the axial direction. In that the locking means is displaceable between the open position and the closed position, the connection of actuating device and surgical instrument can be released again, whereby the rotatability of the second coupling element relative to the second mating coupling element in the first rotational direction as well as the rotatability of the first coupling element relative to the first mating coupling element int the second rotational direction is restored. This facilitates the disassembly of the surgical instrument and the actuating device after the surgical procedure has been completed.

In a first embodiment according to the invention, said second coupling means comprises an L-shaped groove at the distal wire end, and said second mating coupling means comprises an element which engages in said groove when said second coupling means and said second mating coupling means are connected.

Injury to a patient can be minimized by choosing the smallest possible outer diameter of the connector. But the outer diameter of the connector depends on the outer diameter of the wire and the diameter of the second coupling element. If the second coupling means is a groove in the wire, the diameter of the second coupling element is smaller than the diameter of the wire. Therefore, the outer diameter of the connector is determined only by the diameter of the wire. In other words, the second coupling element does not make a larger outer diameter of the connector necessary. The groove thus helps to minimize injury to the patient. The fact that the groove is L-shaped ensures that when the second mating coupling means engages in the groove, the coupling made of them has a good tensile strength. Preferably the element for engaging in the groove is a pin. Advantageously, the second coupling means and the second mating coupling means are additionally frictionally connected to each other. Advantageously, the second coupling means and the second mating coupling means are provided with conical sections that interlock when the second coupling means and the second mating coupling means are connected.

In a further embodiment according to the invention, said L-shaped groove has a depth d₂ in the range of 0.2 mm to 0.5 mm, in the direction of the longitudinal wire axis a length l₂ in the range of 2 mm to 4 mm and in the traverse direction a length b₂ in the range of 1.5 mm to 2 mm.

The depth of the groove depends on the diameter of the wire. However, the diameter of the wire is limited and cannot be increased freely, as otherwise the diameter of the connector would increase, thereby losing the positive effect on minimizing the patient's injury. With a depth of less than d₂ of 0.2 mm the connection of actuating device and surgical instrument withstands only a small tensile force. A depth d₂ of more than 0.5 mm is accompanied by a wire diameter and a connector diameter being too large. A length l₂ of the groove in the direction of the longitudinal wire axis of more than 4 mm complicates the connection of the actuating device and the surgical instrument. A length l₂ of the groove in the direction of the longitudinal wire axis of less than 2 mm makes the correct engagement of second coupling means and second mating coupling means more difficult. The length b₂ of the groove in traverse direction has influences on the stability of the connection between actuating device and surgical instrument. If the length b₂ of the groove in traverse direction is less than 1.5 mm the connection can easily come loose. If the length of the groove in traverse direction is more than 2 mm, the mechanical stability of the wire may be reduced at this local position.

In a further embodiment according to the invention, said first coupling means comprises a L-shaped slot in the distal end of hollow channel, and that said first mating coupling means comprises a component which engages in said slot when the said first coupling means and said first mating coupling means are connected.

Injury to a patient can be minimized by choosing a connector with an outer diameter, being as small as possible. A first coupling means in the form of a L-shaped slot is easy to manufacture, but above all it does not increase the outer diameter of the connector. It is therefore suitable for maintaining the outer diameter of the connector as well as for minimizing injuries to the patient. The L-shaped design of the slot in combination with the component engaged in said slot gives the connection of first coupling means and first mating coupling means a good resistance to tensile forces. Preferably, the component for engaging in the slot is a pin. Preferably, said first coupling means comprises two L-shaped slots located at opposite sides of the connector. Two and more slots help to transfer a tensile force more evenly to the connector. This counteracts wedging of the wire and connector. Advantageously, the first coupling means and the first mating coupling means are additionally frictionally connected to each other. Advantageously, the first coupling means and the first mating coupling means are provided with conical sections that interlock when the first coupling means and the first mating coupling means are connected.

In this context, it has turned out to be advantageous that said L-shaped slot has in the direction of the connector longitudinal axis a length l₁ in the range of 3 mm to 6 mm and in the traverse direction a length b₁ in the range of 1.8 mm to 2.5 mm. The length b₁ is a circumferential length around the outer diameter of the connector.

A slot length l₁ in the direction of the connector longitudinal axis of more than 6 mm demands a large first coupling means and a correspondingly large first mating coupling means. Such large coupling means make minimally invasive surgery more complicated. A slot length l₁ in the direction of the connector longitudinal axis of less than 3 mm is difficult to produce and is associated with low mechanical stability of the connection. A slot length b₁ in the traverse direction of more than 2.5 mm may affect the mechanical stability of the connector at this local position. If the slot length b₁ in the traverse direction is less than 1.8 mm the connection of first coupling means and first mating coupling means may loosen.

In a further embodiment according to the invention, the actuating device has a puncturing section and a locking section, wherein both said wire and said connector have an enlarged diameter in the locking section compared to the puncturing section.

The puncturing section and the locking section of the actuating device differ in their function. While the puncturing section is designed for puncturing the patient's body and should therefore have a diameter as small as possible, the locking section serves to prevent relative rotatability of wire and connector. To ensure that the locking section functions well, other requirements must be met, e.g., it should have an appropriate size so that the locking mechanism is easy to install, mechanically robust and can be repaired if necessary. Therefore, it is advantageous when the locking section components are bigger in size or diameter. Preferably, the locking section is located at the proximal end of the connector. Preferably, the wire diameter in the locking section is in the range of 6 mm to 7 mm. Preferably, the wire diameter in the puncturing section lies in the range of 0.8 mm to 1.5 mm. Preferably, the outer diameter of the connector in the locking section is within the range of 7.5 mm to 9.5 mm, and the outer diameter of the connector in the puncturing section is preferably within the range of 2 mm to 2.5 mm.

In a further embodiment according to the invention said wire is provided with a stop for said locking means which is configured to interact with said locking means when said locking means is in said closed position, and in that said locking means and said stop each have a straight contact side and a rounded contact side, wherein said locking means and said stop are arranged in such a way that said straight contact side of the locking means and said straight contact side of said stop meet, when said wire is rotated in said first rotational direction.

An option to prevent relative rotatability of wire and connector is to provide the wire with a stop that is construed to interact with the locking means in the closed position. The interaction can be undone by moving the locking means to the open position. Due to the fact that the locking means and the stop have different contact sides, they have a different effect depending on the rotational direction. When the wire is rotated in the first rotational direction, the straight contact sides of locking means and stop meet according to the invention. The adjacent surfaces of the straight contact sides prevent further rotation in the first rotational direction. The situation is different when the wire is rotated in the second rotational direction. When the rounded contact sides of the stop and the locking means meet, a rotation is not hindered, because the rounded side of the stop can slide past the locking means by lifting the locking means. The wire remains rotatable in this constellation. It has been found to be advantageous if the locking means in the closed position restricts the rotatability of the wire exclusively in the first rotational direction. This allows the wire to still be rotated in the second rotational direction even with a locking means in the closed position. For example, the wire can be connected to the second mating coupling means of the surgical instrument when the locking means is in the closed position.

Preferably, the wire in the locking section is a profiled wire or a flat wire. Preferably, the stop is created by flattening the wire, e.g., by hammering or by rolling. Preferably, the stop is a bar attached to the wire. Preferably, the wire and bar are one piece. However, the wire and the bar can also be multi-piece. Preferably, the bar is fixed to the wire, e.g., welded or glued to the wire. Preferably, the wire in the puncturing section is a round rod or a wire with a square cross section. Preferably, the wire in the locking section is e.g., a wire with a rectangular cross section, a trapezoidal profile wire, an oval wire or a wire with a half-round cross section. Preferably, the bar has a length in the direction of the longitudinal axis in the range of 30 mm to 50 mm. Preferably, the bar has a width in a direction perpendicular to the longitudinal axis in the range of 5 mm to 10 mm.

In a further embodiment according to the invention, said actuating device comprises a lever for moving said locking means between said open position and said closed position, wherein said locking means is fixed to a first arm of said lever.

A lever can be operated with one hand; it is easy and quick to use. Preferably, the locking means can be moved out of the hollow channel by pressing the lever.

It has turned out to be advantageous, when said lever comprises a second lever arm and a spring element arranged on said second lever arm, being configured to keep said locking means in said closed position when said lever is not actuated.

The spring element keeps the lever and thus the locking means in the closed position when the lever is not actuated. This facilitates the operation of the actuating device, as the lever needs only to be actuated when the locking means is to be transferred to the open position, e.g., when the surgical instrument is mounted, changed or removed.

In a further embodiment according to the invention said actuating device comprises a handle and an adjusting wheel for rotating said connector along said connector longitudinal axis, wherein said adjusting wheel comprises an opening and a blocking means, said blocking means being movable between a non-locking position and a locking position, and said blocking means being configured to engage said opening when said blocking means is moved to said locking position, whereby in the locking position said handle is fixed to said adjusting wheel.

During a surgical procedure, it is often necessary to be able to precisely align the tool in the patient's body. For this purpose, known surgical systems have an adjusting wheel by which the connector can be rotated, the adjusting wheel being rotatable relative to the handle. However, when mounting the surgical instrument to the actuating device, the rotatability of the handle makes it more difficult to mount the surgical instrument. It is therefore advantageous if the handle can be fixed to the adjusting wheel in a rotationally fixed manner by means of a blocking means. In this way, the connector can be rotated by rotating the handle. By releasing the blocking means, the rotatability of the handle and the adjusting wheel relative to each other can be restored. Preferably, the handle is attached to the adjusting wheel by engaging the blocking means in an opening of the adjusting wheel. Preferably, said opening is a toothed rim and said blocking means is a pin or bolt.

In a further embodiment according to the invention said surgical instrument comprises a first instrument part and a second instrument part, said first instrument part and said second instrument part being rotationally fixed, but movable relative to each other by a translatory movement, wherein said first instrument part is provided with said first mating coupling means and said second instrument part is provided with the second mating coupling means.

Due to the arrangement of the first instrument part and the second instrument part as described above, they can be moved relative to each other by pulling or releasing the wire. Such a movement can be used for the control of certain surgical instruments, e.g., scissors, clamps or for the actuation of a cautery.

With regard to the actuating device for actuating a surgical instrument, the above-mentioned objective is achieved in that said second coupling means is configured to be connectable to said second mating coupling means by rotation of said wire about said wire longitudinal axis in a second rotational direction opposite to said first rotational direction and in that the actuating device comprises a locking means which is movable between an open position and a closed position, the open position permitting relative rotatability in the axial direction, and the closed position preventing relative rotatability of wire and connector when said first coupling means is connected to said first mating coupling means and said second coupling means is connected to said second mating coupling means.

In known surgical systems, the connection of the surgical instrument to the actuating device in the patient's body may be complex and time-consuming. The present invention is based on the idea of simplifying and improving the connection of actuating device and surgical instrument.

According to the present invention, the wire is allocated to the actuating device. This means that the wire has not to be fed back through the connector to be connected to the actuating device. However, this means that not only the connector but also the wire must be connectable to a surgical instrument in the patient's body. Therefore, the wire is provided with a second coupling means configured to be connected to a second mating coupling means on a surgical instrument. Both the first coupling means and the second coupling means are connectable to their mating coupling means by a rotation. Such a rotational connection is easy to create in the patient's body.

However, in order to prevent unintentional loosening of the connection of actuating device and surgical instrument, the actuating device is configured such that the first coupling means of the connector is connectable to the first mating coupling means on the surgical instrument by rotation of the connector about the connector longitudinal axis in a first rotational direction. Furthermore, the wire has a second coupling means which is connectable to a second mating coupling means by rotation of the wire about the wire longitudinal axis in a second rotational direction opposite to the first rotational direction. Furthermore, according to the invention the actuating device comprises a locking means preventing relative rotatability of wire and connector when the first coupling means is connected to the first mating coupling means and the second coupling means is connected to the second mating coupling means.

Furthermore, the locking means can be moved to an open position, in which the rotatability of the second coupling element relative to the second mating coupling element in the first rotational direction as well as the rotatability of the first coupling element relative to the first mating coupling element int the second rotational direction is restored. This facilitates the disassembly of the actuating device and a surgical instrument after the surgical procedure has been completed.

With regard to the surgical instrument, the above-mentioned objective is achieved in that the surgical instrument is configured for use in a surgical system according to one or more of the claims 1 to 12, or for use with an actuating device according to claim 13.

### DEFINITIONS

A *surgical instrument* according to the invention is a tool or device suitable for performing surgery on a patient, e.g., a clamp, a gripper, an electrode, a clip applier, a grasper, surgical scissors, a retractor, a tip, a cautery, a litigation loop, a distractor, a suction and/or irrigation device, a light source and/or a suturing device.

An *actuating device for a surgical instrument* within the meaning of the invention is a device suitable for controlling and operating the surgical instrument. It has a first section for insertion in to the patient's body, e.g., a connector, and a second section with control elements for the control of the surgical instrument, the second section remaining outside the patient's body. The actuating device is designed for extra corporal controlling the surgical instrument.

A *connector* according to the invention represents the connecting element by which the surgical instrument and the actuating device are connected to each other. During surgery, the connector may be arranged fully or partially in the patient's body. The connector usually is hollow cylindrical in shape, having a hollow channel which surrounds a wire, the wire being movable in the direction of the longitudinal axis of the connector.

A *wire* according to the invention is a flexible or non-flexible, solid wire or cable, suitable for transmitting mechanical force or energy by its linear movement relative to its housing, e.g., the connector. Usually, the transmitted mechanical force is a pulling force being transmitted by the wire to the surgical instrument, thereby controlling the surgical instrument.

A *coupling means* according to the invention is a mechanical device construed to interact with another coupling means and suitable for creating a connection between devices, e.g., the surgical instrument and the actuating device, wherein the coupling means is connectable to a mating coupling means.

The term *"connection of actuating device and surgical instrument"* is used in some sections of the present application to simplify the wording. The term describes the effect, namely the actuating device and surgically instrument being connected, without mentioning all the components being involved therein, e.g. first coupling means, second coupling means, first mating coupling means, etc.

### DETAILED DESCRIPTION

The present invention will be more apparent from the following description of embodiments with reference to the accompanying drawings, in which:
- **Figure 1**: is a side view and top view of a first surgical system according to the invention, comprising a surgical instrument and an actuating device for actuating the surgical instrument,
- **Figure 2**: schematically depicts in a perspective view an end section of a connector with a wire arranged in the hollow channel of the connector, wherein the connector is provided with a first coupling means and the wire is provided with a second coupling means,
- **Figure 3**: shows schematically in a sectional view the connection of an actuating device and a surgical instrument according to the invention,
- **Figure 4**: presents a sectional view of an actuating device according to the invention, having a connector, a wire, a handle, an adjusting wheel and a blocking means for fixing the handle to the adjusting wheel, as well as a lever for moving a locking means, with which the relative rotatability of the connector and the wire is controllable,
- **Figure 5**: shows in perspective view a section of an actuating device according to the invention having a handle, a connector, an adjusting wheel for the connector and a blocking pin for fixing the handle to the connector,
- **Figure 6**: is a schematic cross-section through the locking section of an actuating device according to the invention in the closed position, illustrating the interaction of locking means and stop, and
- **Figure 7**: a schematic representation of a second surgical system according to the invention.

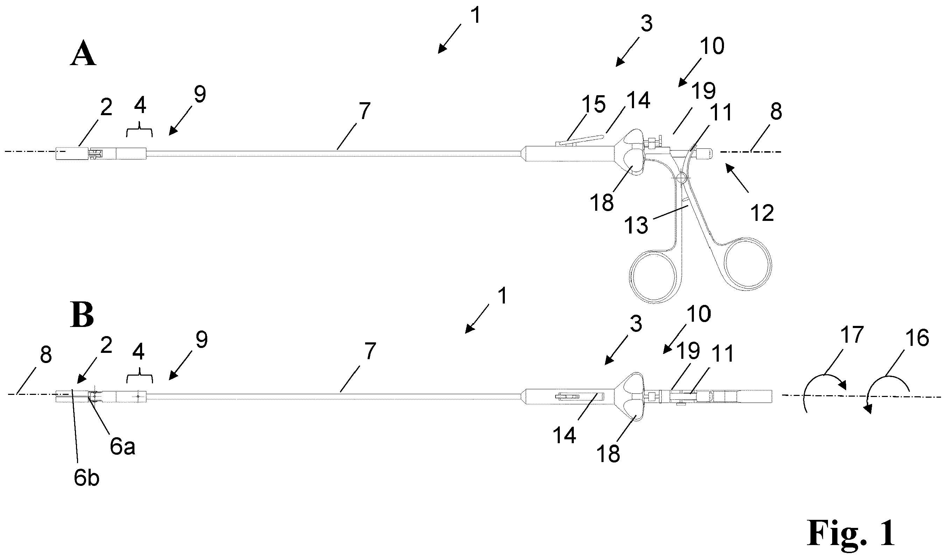

Where reference numbers in Figures 1 to 7 are the same, they refer to identical oder equivalent components and parts.

**Figure 1** shows a schematic representation of a first embodiment of a surgical system according to the invention, which is assigned the reference number 1. Figure 1A shows a side view of the surgical system 1 and Figure 1B a top view of the surgical system 1.

The surgical system 1 comprises a surgical instrument 2 and an actuating device 3 for actuating the surgical instrument 2. The surgical instrument 2 is a tissue clamp that can be used to capture and hold tissue or material. The tissue clamp comprises two grippers 6a, 6b. The surgical instrument 2 has first and a second mating coupling means, being connectable to a first coupling means or a second coupling means of the actuating device 3, respectively. In the Figures 1A and 1B the first and the second coupling means as well as the first mating coupling means and the second mating coupling means are not shown; they are located in the connection area 4, which is marked by a bracket. The connection area 4 has a round cross-section with a diameter a of about 5 mm.

The actuating device 3 comprises a connector 7 having a connector longitudinal axis 8. The connector 7 is shaped like a hollow cylinder and encloses a hollow channel (not shown). The hollow channel extends along the longitudinal axis 8 between a distal end 9 and a proximal end 10. The distal end 9 is provided with a first coupling means (not shown), being configured to be coupled to the first mating coupon means (not shown) on the surgical instrument 2. The outer diameter of the connector 7 is 2.5 mm. The diameter of the hollow channel is 2.3 mm. The connector 7 is made of surgical stainless steel.

The hollow channel (not shown) guides a wire 11, being a solid wire made of surgical stainless steel. The wire 11 has a longitudinal wire axis which is not shown separately in Figure 1 because it is essentially coaxial to the connector longitudinal axis 8. The wire 11 has distal wire end (not shown) being provided with a second coupling means (not shown), which is connected to a second mating coupling means (not shown) on the surgical instrument 2, both located in the connection area 4 as well as a proximal wire end 12 which is fixed to the handle 13 of the actuating device 3.

The connection of actuating device 3 and surgical instrument 2 in the connection area 4 will be described in detail with reference to the following figures. But at this point, it should be said that the connector 7 is coupled to the surgical instrument 2 by rotation of the connector 7 about the connector longitudinal axis 8 in a first rotational direction 16. The wire 11, however, is coupled to the surgical instrument 2 by rotation of the wire 11 about the wire longitudinal axis (which is essentially coaxially to the connector longitudinal axis 8) in the opposite direction, namely in the second rotational direction 17.

Furthermore, the actuating device 3 comprises a lever 14 with which a locking means 15 is movable into the hollow channel of the actuating device 3, thereby preventing relative rotatability of the wire 11 relative to the connector 7 in the first rotational direction 16. Due to the interaction of the mounting points, namely first coupling means and first mating coupling means as well as second coupling means and second mating coupling means on the one hand and locking means 15 on the other hand, the actuating device 3 is connected to the surgical instrument 2 in a rotationally fixed manner.

Connecting the surgical instrument 2 and the actuating device 3 is easier when the connector 7 is connected to the handle 13 in a rotationally fixed manner. This allows the connection between the connector 7 and the surgical instrument 2 to be made by turning the handle 13. On the other hand, the possibility to rotate the handle 13 and connector 7 relative to each other during the operation facilitates the precise alignment of the surgical instrument 2. To take advantage of both of these benefits, the actuating device 3 is equipped with an adjusting wheel 18 and a blocking means 19. The blocking means 19 can assume a non-locking position, in which the handle 13 and the connector 7 can be rotated relative to each other e.g., by turning the adjusting wheel 18, and a locking position, in which the handle 13 is fixed to the connector 7.

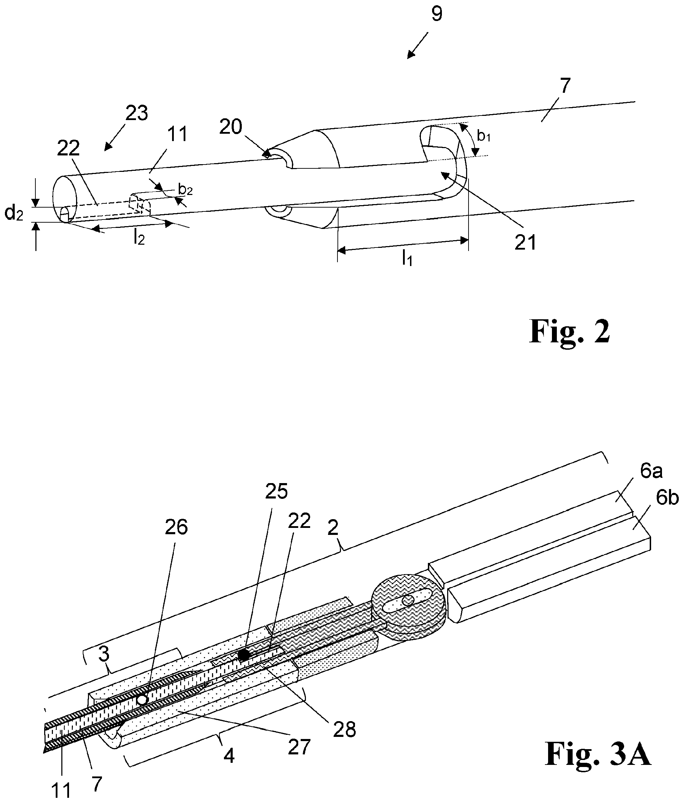

**Figure 2** schematically shows in an enlarged view the distal end 9 of the connector 7 shown in Figure 1, having a hollow channel 20. The connector 7 is provided with a first coupling means 21 comprising two L-shaped slots. Of the two slots, only one slot is seen in Figure 2. The other slot (not shown) is on the opposite side of the connector 7, being designed accordingly. The slots are configured to be each coupled to a corresponding pin on a surgical instrument, thereby forming a bayonet mount.

A wire 11 is arranged in the hollow channel 20. The wire 11 has an outer diameter of 1 mm and is made of surgical stainless steel. The distal wire end 23 is provided with a second coupling means 22 which is configured to be connected to a second mating coupling means on the surgical instrument. The second coupling means 22 is a L-shaped groove which corresponds to a pin or bolt on the surgical instrument to be connected, thereby forming a bayonet mount. The L-shaped groove has in the direction of the longitudinal wire axis a length l₂ of 3 mm, a depth d₂ of 0.35 mm and in the transverse direction a length b₂ of 1.6 mm. The length b₂ is a circumferential length around the outer diameter of the wire.

**Figure 3A** shows schematically in a sectional view the connection of the actuating device 3 and the surgical instrument 2 shown in Figure 1. The surgical instrument 2 is a tissue clamp that can be used to capture and hold tissue or material. The tissue clamp comprises two grippers 6a, 6b.

The surgical instrument 2 has a first receptacle 27 for the connector 7 and a second receptacle 28 for the wire 11. The second receptacle 28 has a pin 25 which engages in the groove 22 of the wire 11 when connected. The first receptacle 27 has two pins. These are not actually visible in Figure 1, as the pins 11 and 26 are arranged on top and bottom of the connector 7 at an angle of 180° to each other. However, their position is indicated by point 26. Each of the pins 26 engages in a slot (not shown) of the connector 7.

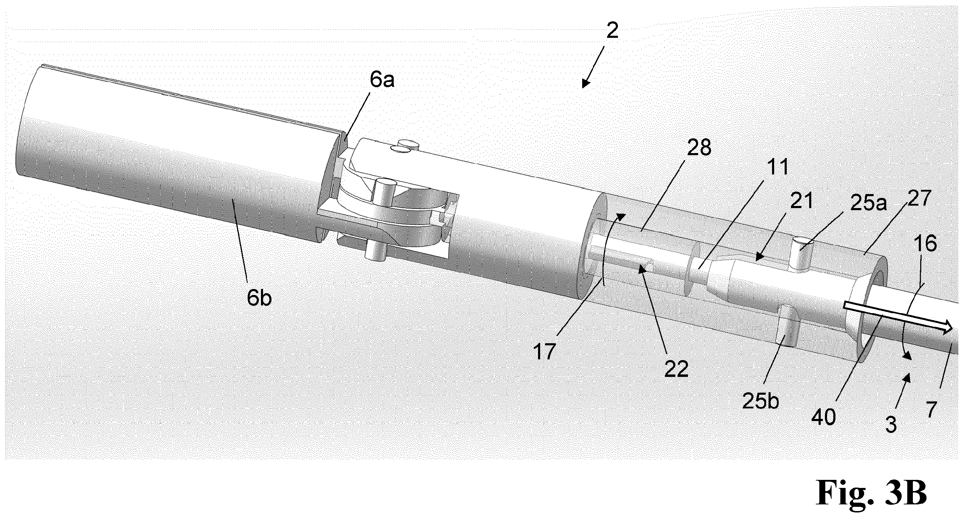

**Figure 3B** shows the connection shown in Figure 3A of the actuating device 3 and the surgical instrument 2 in a different, three-dimensional and photo-like representation. Where identical reference numbers are used in Figures 3A and 3B, these designate identical or equivalent components.

Actuating device 3 and surgical instrument 2 are connected to each other by inserting the connector 7 into the first receptacle 27 of the actuating device 2 and rotating the connector 7 about the connector longitudinal axis in the first rotational direction 16. This results in the two pins 25a, 25b engaging in the L-shaped first coupling means 21 of the connector 7. The rotation ends automatically when the end of the traverse section of the L-shaped first coupling means is reached.

Then the wire 11 of the actuating device 3 is connected to the surgical instrument 2 by inserting the end of wire 11 in the second receptacle 28 of the surgical instrument 2 and rotating the wire 11 about the wire longitudinal axis in the second rotational direction 17. This results in a pin (not shown) of the second receptacle 28 being inserted into the groove 22 of the wire. The L-shape of the groove 22 prevents further rotation in the second rotational direction 17.

Applying a force to the wire 11 in the direction 40 of the wire longitudinal axis causes the wire 11 to move relative to the connector 7 in the direction 40 of the wire longitudinal axis. As the wire 11 is attached to the surgical instrument 2, namely the tissue clamp, the relative movement of the wire 11 relative to the connector 7 in the direction 40 of the wire longitudinal axis results in the two grippers 6a, 6b of the tissue clamp being moved apart.

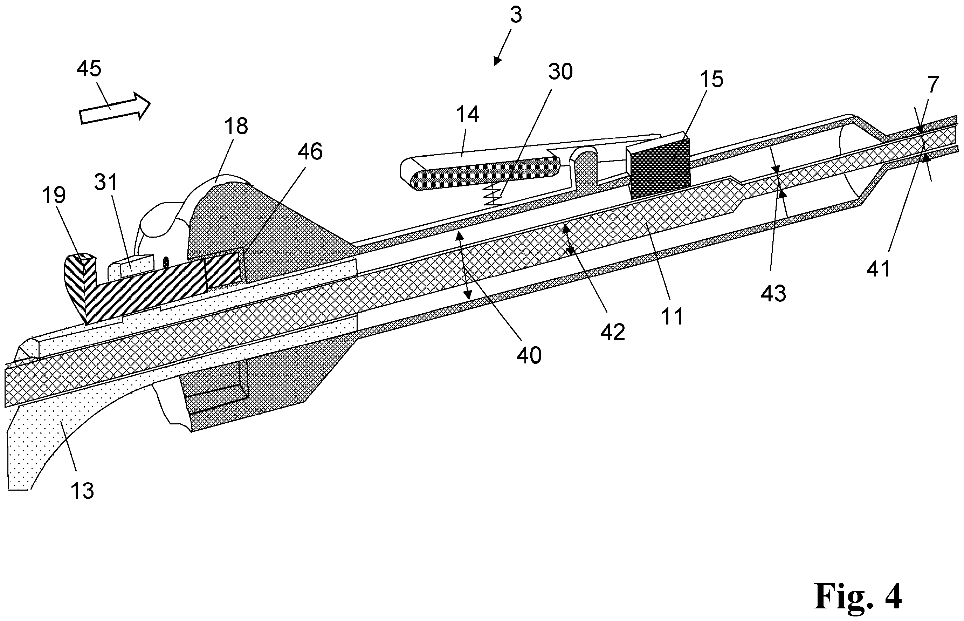

**Figure 4** shows a cross-section through an actuating device according to the invention to which the reference number 3 is assigned. The actuating device 3 comprises a connector 7, the distal end of which is connectable to a surgical instrument (not shown). Figure 4 illustrates the proximal end of the actuating device 3, in particular the locking section in which the wire 11 and the connector 7 are rotationally fixable to each other.

The non-rotational fixation of wire 11 and connector 7 is facilitated if wire 11 and connector 7 have larger diameter in the locking section compared to the puncturing section at the distal end of the hollow channel. The wire 11 is created by flattening. The maximum wire diameter in the locking section 42 is 6.5 mm; the maximum wire diameter in the puncturing section 43 is 1 mm. The inner diameter of the connector 7 in the locking section 40 is 8.5 mm, the inner diameter of the connector 7 in the puncturing section 41 is 1.4 mm. The connector 7 is made of surgical stainless steel; it has a wall thickness of 0.4 mm.

The adjusting wheel 18 is connected to the connector 7; they are made of one piece. Thus, each rotation of the adjusting wheel 18 is associated with a rotation of the connector 7. Without special measures, the wire 11 and the connector 7 are rotatable relative to each other. Therefore, a rotation of the adjusting wheel 18 would lead to a rotation of the connector 7 relative to the wire 11. However, since both the wire 11 and the connector 7 are connected to the surgical instrument by rotation, a rotation of the connector 7 and the wire 11 relative to each other could result in loosening the connection of surgical instrument 2 and connector 7 or wire 11, respectively. Therefore, the actuating device 3 comprises a locking means 15 which is able to prevent the free rotation of wire 11 and connector 7 relative to each other. The locking means 15 is a cuboid with one rounded edge. It has a length of 35 mm, a width of 0.5 mm and a height of 6 mm; it can assume a closed position and an open position via lever 14. The lever 14 has a spring 30 that causes the locking means 15 to be held in the closed position. In the closed position, the locking means 15 protrudes in the hollow channel so deeply that it contacts one side of the flattened wire 11 such that it stops the rotation of the wire 7 in the first direction of rotation, opposite to the direction in which the wire 11 is connectable to the second mating coupling means of a surgical element.

The handle 13 and the adjusting wheel 18 are mounted so that they can rotate relative to each other. However, this makes it difficult to connect the actuating device 3 with the surgical instrument 2, because in this case the connection between the actuating device 3 and the surgical instrument 2 can only be made by turning the adjusting wheel 18 which is a small component and therefore more difficult to operate. Therefore, the adjusting wheel 18 and the handle 13 are provided with a blocking means 19, with which the handle 13 and the adjusting wheel 18 can be firmly connected. On the other hand, it is important for the exact alignment of the surgical instrument 2 during operation that the handle 13 and the adjusting wheel 18 are movable relative to each other. Therefore, the blocking means 19 is movable between a locking position in which adjusting wheel 18 and handle 13 are locked to each other and a non-locking position in which adjusting wheel 18 and handle 13 can freely rotate relative to each other. When the blocking means 19 is moved in the direction of arrow 45, it engages in an opening 46 of the adjusting wheel 18, thereby fixing handle 13 and adjusting wheel 18. Moving the blocking means 19 in the opposite direction to the direction of arrow 45 restores the relative movability of the adjusting wheel 18 and the handle 13.

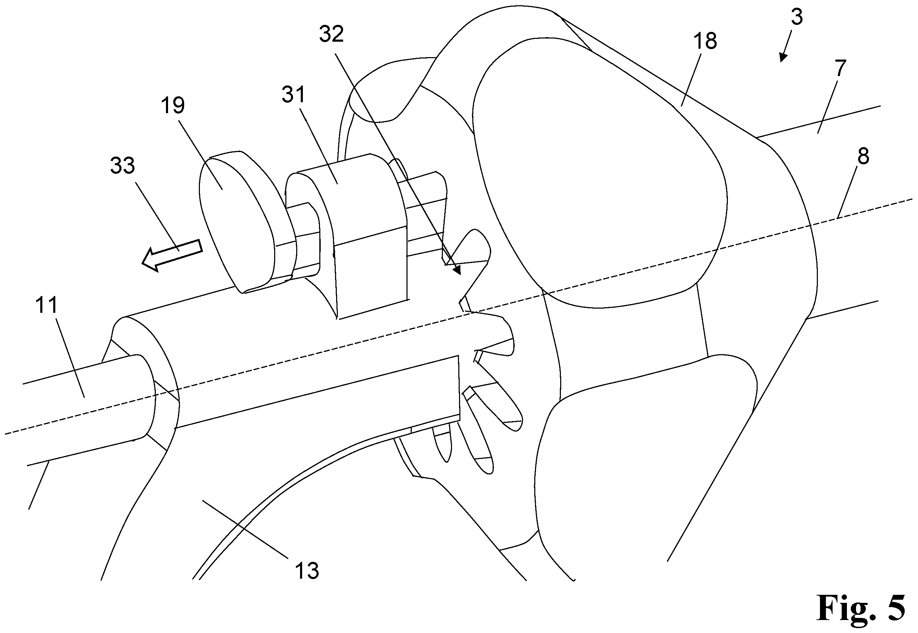

**Figure 5** shows the proximal end of the actuating device 3 in detail. The actuating device 3 comprises a connector 7, a wire 11 guided in the hollow channel of the connector 7 and a handle 13. The connector 7 is equipped with an adjusting wheel 18 which is connected to the connector 7 in such a way that the adjusting wheel 18 can only be rotated along the connector longitudinal axis 8 together with the connector 7.

The handle 13, however, is a separate part that can in principle rotate freely along the connector longitudinal axis 8 in relation to the connector 7 and the adjusting wheel 18.

However, the actuating device 3 has a blocking mechanism with which the handle can be fixed to the adjusting wheel 18 and the connector 7, respectively. For this purpose, the adjusting wheel 18 has an opening in the form of a toothed rim 32 and the handle 13 has a locating channel 31 for a blocking means 19 in form of locating pin. When the locating pin engages in the toothed rim, the handle 13 and the adjusting wheel 18 fixed to each other so that the handle 13, the adjusting wheel 18 and the connector 7 can only be moved together. The connection between handle 13 and adjusting wheel 18 can be released by pulling the locking pin out of the toothed rim in the direction of the arrow 34.

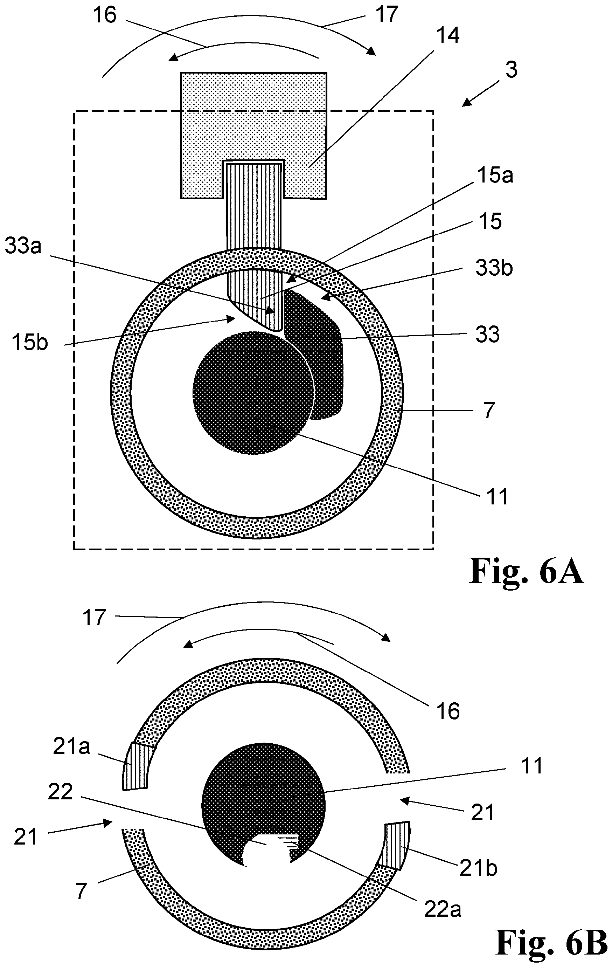

**Figures 6A and 6B** are a schematic illustration for explaining the operation of the connection between the actuating device 3 and the surgical instrument 2. Figure 6A shows a simplified cross-section through the locking section of the actuating device 3. Figure 6B depicts a simplified sectional view of the distal end of wire 11 and connector 7. Figures 6A and 6B have the same direction of view, namely towards the distal end of the connector 7. In detail:
Figure 6A shows a simplified representation of:
   - a connector 7 enclosing a hollow channel extending along a connector longitudinal axis (not shown) perpendicular to the drawing plane,
   - a wire 11 with a longitudinal wire axis essentially parallel to the connector longitudinal axis,
   - a stop 33 attached to the wire 11 by welding, having a straight contact side 33a and a rounded contact side 33b,
   - a locking means 15 being movable by actuation of lever 14 between a closed position (shown in Figure 6A) and an open position in which locking means 15 and stop 33 are not in contact with each other (not shown in Figure 6A), the locking means 15 having a straight contact side 15a and a rounded contact side 15b.
Figure 6B shows schematically
   - the distal end of the connector 7 having two first coupling means 21, namely two L-shaped slots, whereby the position of the section of the L-shaped slots perpendicular to the connector longitudinal axis is indicated by hatched areas 21a, 21b, and
   - the distal wire end of wire 11, having a second coupling means 22, namely a L-shaped groove 22 with section parallel to the wire longitudinal axis and a section perpendicular to the wire longitudinal axis (22a).

The method for connecting and disconnecting the surgical instrument and the actuating device is described below in more detail.
1. The locking means 15 is in closed position.
a. The slots of connector 7 are connected to a first mating coupling (not shown) of the surgical instrument by rotating the connector 7 in the first direction of rotation 16. The connector 7 rotates together with the wire 11, because the straight contact sides 15a, 33a of stop 33 and locking means 15 meet when the connector 7 rotates in the first rotational direction 16. The rotation ends automatically when the end of the traverse section of the L-shaped slot is reached.
b. The groove of wire 11 is connected to a second mating coupling (not shown) of the surgical instrument by rotating the wire 11 in the second rotational direction 17. In this case, the rounded contact sides 15b, 33b of the stop 33 and the locking means 15 meet. The rounded contact sides 15b, 33b cannot hinder the turning movement in the second direction of rotation 17, since the stop 33 can - due to its shape - lift the locking means 15 by itself. Consequently, the locking means 15 and the stop 33 allow the wire 11 to rotate freely in the second direction of rotation 17. This permits to hold the connector 7 in its connection position with the surgical instrument, while at the same time the wire 11 can be rotated in the second direction of rotation 17 until it is itself connected to the surgical instrument and the L-shaped groove prevents further rotation in the second rotational direction 17.
As the following table shows, the wire 11 and the connector 7 are rotationally fixed to each other:

**Table 1**

| Rotation in ... | Wire 11 | Connector 7 |
|---|---|---|
| ... first rotational direction 16 | Rotation blocked by contact of stop 33 with locking means 15. | Rotation blocked by first coupling means 21 (slots) |
| ... second rotational direction 17 | Rotation blocked by second coupling means 22 (groove) | Rotation blocked by contact of locking means 15 with stop 33. |

2. When the surgical instrument is not needed any more, the locking means 15 is moved to the open position by pressing the lever 14, thereby enabling the disassembly of the surgical instrument and the actuating device.

## Claims

1. A surgical system (1; 100), comprising
- a surgical instrument (2; 102), and
- an actuating device (3; 103) for actuating the surgical instrument (2; 102), the actuating device (3; 103) comprising a connector (7; 107) having a connector longitudinal axis (8; 108) in which a hollow channel (20; 120) extends along the longitudinal axis between a distal end (9; 109) and a proximal end (10; 110),
- wherein the distal end (9; 109) is provided with a first coupling means (21) configured to be coupled to a first mating coupling means on the surgical instrument (2; 102),
- wherein said hollow channel (20; 120) axially movably receives a wire having a longitudinal wire axis parallel with the connector longitudinal axis (8; 108), and said wire having a distal wire end (23; 123) and a proximal wire end (12; 112), said distal wire end (23; 123) being provided with a second coupling means (22) configured to be connected to a second mating coupling means on the surgical instrument (2; 102),
- said first coupling means (21) being configured to be connectable to said first mating coupling means by rotation of said connector (7; 107) about said connector longitudinal axis (8; 108) in a first rotational direction (16), **characterized in that**
- said second coupling means (22) is configured to be connectable to said second mating coupling means by rotation of said wire about said wire longitudinal axis in a second rotational direction (17) opposite to said first rotational direction (16),
- and **in that** the actuating device (3; 103) comprises a locking means (15; 115) which is movable between an open position and a closed position, the open position permitting relative rotatability of wire and connector (7; 107) in the first rotational direction and the second rotational direction and relative displaceability in the axial direction, and the closed position preventing relative rotatability of wire and connector (7; 107) when said first coupling means (21) is connected to said first mating coupling means and said second coupling means (22) is connected to said second mating coupling means.

2. A surgical system (1; 100) according to claim 1, **characterized in that** said second coupling means (22) comprises an L-shaped groove at the distal wire end (23; 123), and that said second mating coupling means comprises an element which engages in said groove when said second coupling means (22) and said second mating coupling means are connected.

3. A surgical system (1; 100) according to claim 2, **characterized in that** said L-shaped groove has a depth (d₂) in the range of 0.2 mm to 0.5 mm, in the direction of the longitudinal wire axis a length (l₂) in the range of 2 mm to 4 mm and in the traverse direction a length (b₂) in the range of 1.5 mm to 2 mm.

4. A surgical system (1; 100) according one of the preceding claims, **characterized in that** said first coupling means (21) comprises a L-shaped slot in the distal end (9; 109) of hollow channel (20; 120), and that said first mating coupling means comprises a component which engages in said slot when the said first coupling means (21) and said first mating coupling means are connected.

5. A surgical system (1; 100) according to claim 4, **characterized in that** said L-shaped slot has in the direction of the connector longitudinal axis (8; 108) a length (l₁) in the range of 3 mm to 6 mm, and in the traverse direction a length (bi) in the range of 1.8 mm to 2.5 mm.

6. A surgical system (1; 100) according to one of the preceding claims, **characterized in that** the actuating device (3; 103) has a puncturing section and a locking section, wherein both said wire and said connector (7; 107) have an enlarged diameter in the locking section compared to the puncturing section.

7. A surgical system (1; 100) according to one of the preceding claims, **characterized in that** said wire is provided with a stop (33; 133) for said locking means (15; 115) which is configured to interact with said locking means (15; 115) when said locking means (15; 115) is in said closed position, and **in that** said locking means (15; 115) and said stop (33; 133) each have a straight contact side (15a; 33a) and a rounded contact side (15b; 33b), wherein said locking means (15; 115) and said stop (33; 133) are arranged in such a way that said straight contact side of the locking means (15; 115) and said straight contact side of said stop (33; 133) meet, when said wire is rotated in said first rotational direction.

8. A surgical system (1; 100) according to one of the preceding claims, **characterized in that** said actuating device (3; 103) comprises a lever (14) for moving said locking means (15; 115) between said closed position and said open position, wherein said locking means (15; 115) is fixed to a first arm of said lever (14).

9. A surgical system (1; 100) according to claim 8, **characterized in that** said lever (14) comprises a second lever arm and a spring element (30) arranged on said second lever arm, being configured to keep said locking means (15; 115) in said closed position when said lever (14) is not actuated.

10. A surgical system (1; 100) according to one of the preceding claims, **characterized in that** said actuating device (3; 103) comprises a handle (13) and an adjusting wheel (18) for rotating said connector (7; 107) along said connector longitudinal axis (8; 108), wherein said adjusting wheel (18) comprises an opening and a blocking means (19), said blocking means (19) being movable between a non-locking position and a locking position, and said blocking means (19) being configured to engage said opening when said blocking means (19) is moved to said locking position, whereby in the locking position said handle (13) is fixed to said adjusting wheel (18).

11. A surgical system (1; 100) according to claim 10, **characterized in that** said opening is a toothed rim (32) and **in that** said blocking means (19) is a pin or bolt.

12. A surgical system (1; 100) according to one of the preceding claims, **characterized in that** said surgical instrument (2; 102) comprises a first instrument part (171) and a second instrument part (170), said first instrument part (171) and said second instrument part (170) being rotationally fixed, but movable to each other by a translatory movement, wherein said first instrument part (171) is provided with said first mating coupling means (126) and said second instrument part (170) is provided with the second mating coupling means.

13. An actuating device (3; 103) for actuating a surgical instrument (2; 102), the actuating device (3; 103) comprising a connector (7; 107) having a connector longitudinal axis (8; 108) in which a hollow channel (20; 120) extends along the longitudinal axis between a distal end (9; 109) and a proximal end (10; 110), wherein the distal end (9; 109) is provided with a first coupling means (21) configured to be coupled to a first mating coupling means on the surgical instrument (2; 102),
wherein said hollow channel (20; 120) axially movable receives a wire having a longitudinal wire axis parallel with the connector longitudinal axis (8; 108), and said wire having a distal wire end (23; 123) and a proximal wire end (12; 112), said distal wire end (23; 123) being provided with a second coupling means (22) configured to be connected to a second mating coupling mean on the surgical instrument, said first coupling means (21) being configured to be connectable to said first mating coupling means by rotation of said connector (7; 107) about said connector longitudinal axis (8; 108) in a first rotational direction (16), **characterized in that**
- said second coupling means (22) is configured to be connectable to said second mating coupling means by rotation of said wire about said wire longitudinal axis in a second rotational direction (17) opposite to said first rotational direction (16)
- and **in that** the actuating device (3; 103) comprises a locking means (15; 115) which is movable between an open position and a closed position, the open position permitting relative rotatability in the axial direction, and the closed position preventing relative rotatability of wire and connector (7; 107) when said first coupling means (21) is connected to said first mating coupling means and said second coupling means (22) is connected to said second mating coupling means.

14. A surgical instrument (2; 102) configured for use in a surgical system (1; 100) according to one or more of the claims 1 to 12, or for use with an actuating device (3; 103) according to claim 13.
